(19) Europäisches Patentamt

European Patent Office

Office européen des brevets

(11)  EP 0 400 330 B2

(12)  **NEUE EUROPÄISCHE PATENTSCHRIFT**

(45) Veröffentlichungstag und Bekanntmachung des Hinweises auf die Entscheidung über den Einspruch:
07.03.2001  Patentblatt 2001/10

(45) Hinweis auf die Patenterteilung:
10.03.1993  Patentblatt 1993/10

(21) Anmeldenummer: 90108017.6

(22) Anmeldetag: 27.04.1990

(51) Int Cl.[7]: **A61K 7/13**

(54)  **Oxidationshaarfärbemittel**

Oxidation hair dyes

Colorants d'oxydation pour cheveux

(84) Benannte Vertragsstaaten:
**CH DE ES IT LI NL SE**

(30) Priorität: **27.05.1989  DE 3917304**

(43) Veröffentlichungstag der Anmeldung:
**05.12.1990  Patentblatt 1990/49**

(73) Patentinhaber: **Wella Aktiengesellschaft**
**64295 Darmstadt (DE)**

(72) Erfinder:
• **Mager, Herbert, Dr.**
  **CH-1723 Marly (CH)**
• **Braun, Hans-Jürgen, Dr.**
  **CH-3182 Überstorf (CH)**
• **Rau, Robert, Dr.**
  **D-6112 Gross-Zimmern (DE)**
• **Clausen, Thomas, Dr.**
  **D-6146 Alsbach (DE)**
• **Konrad, Eugen**
  **D-6100 Darmstadt (DE)**

(74) Vertreter: **Meyer-Dulheuer, Karl-Hermann, Dr.**
**Meyer-Dulheuer & Ackermann**
**Stiftstrasse 2**
**60313 Frankfurt am Main (DE)**

(56) Entgegenhaltungen:
**EP-A- 0 007 537**      **EP-A- 0 166 155**
**EP-A- 0 258 586**      **WO-A-88/00823**
**DE-A- 3 430 513**      **DE-A- 3 545 371**
**DE-A- 3 627 398**      **GB-A- 2 163 154**
**GB-A- 2 168 727**

• **K. Schrader "Grundlagen und Rezepturen der Kosmetika", Hüthig Verlag, 1979, S. 536-541**

Bemerkungen:
Die Akte enthält technische Angaben, die nach dem Eingang der Anmeldung eingereicht wurden und die nicht in dieser Patentschrift enthalten sind.

EP 0 400 330 B2

**Beschreibung**

Gegenstand der Erfindung sind Mittel zur oxidativen Färbung von Haaren mit einem Gehalt an einer nicht sensibilisierenden, nicht mutagenen Entwicklersubstanz-Kupplersubstanz-Kombination, wobei 2-(2,5-Diaminophenyl) ethanol als Entwicklersubstanz in Kombination mit bestimmten Kupplersubstanzen verwendet wird.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels.

Die derzeit bevorzugt eingesetzten Entwicklersubstanzen sind 2,5-Diaminotoluol, p-Phenylendiamin, 4-Aminophenol und 4-Amino-3-methylphenol, während als Kupplersubstanzen eine Vielzahl von Verbindungen, wie z. B. Resorcin, 2-Methylresorcin, 4-Chlorresorcin, 2-Chlorresorcin, 4,6-Dichlorresorcin, 2-Amino-4-(2-hydroxyethyl)aminoanisol, 2-(2,4-Diaminophenoxy)ethanol, 1-Naphthol, m-Phenylendiamin, 5-Amino-2-methylphenol, 3-Amino-4-chlor-6-methylphenol, 3-Amino-2-methylphenol, 2-(4-Amino-2-hydroxyphenoxy)ethanol, 4-Amino-1,2-methylendioxybenzol, 2,4-Diamino-5-ethoxytoluol, 4-Hydroxyindol, 3-Amino-5-hydroxy-2,6-dimethoxypyridin, 3,5-Diamino-2,6-dimethoxypyridin, 3-Amino-6-methoxy-2-methylamino-pyridin, Thymol, 2-(2-Hydroxyethyl)-5-methylphenol, 2-Hydroxymethyl-5-methylphenol,2-Hydroxymethyl-5-methylphenol, 3-Aminophenol und N-(2-Hydroxyethyl)-3,4-methylendioxyanilin, verwendet werden.

An Oxidationsfarbstoffe, die zur Färbung menschlicher Haare verwendet werden sollen, werden zahlreiche besondere Anforderungen gestellt. Die Farbstoffe müssen in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert.

In jedem Fall müssen solche Haarfärbungen ohne Einwirkung von Licht, Reibung und chemischen Mitteln über einen Zeitraum von mindestens vier bis sechs Wochen stabil bleiben. Ausserdem ist es erforderlich, dass durch Kombination geeigneter Entwickler- und Kupplerkomponenten eine breite Palette verschiedener Farbnuancen erzeugt werden kann.

Gegen die zur Zeit hauptsächlich eingesetzten Entwicklersubstanzen p-Phenylendiamin, 2,5-Diaminotoluol und 4-Aminophenol wurden in letzter Zeit Bedenken bezüglich ihrer physiologischen Verträglichkeit erhoben. Ein Problem stellt dabei die Sensibilisierung bestimmter Personen auf p-Phenylendiaminderivate dar (Paraallergiker). Diesem Personenkreis war es bisher nicht möglich, sich die Haare mit Haarfärbemitteln auf der Basis von p-Phenylendiaminderivaten färben zu lassen. Weiterhin ist nicht nur die sensibilisierende Wirkung der reinen, Entwicklerubstanzen, sondern auch die der Mischungen mit Kupplersubstanzen sowie die der daraus gebildeten Farbstoffe zu berücksichtigen.

Mit der Bereitstellung von Pyrimidinderivaten als Entwicklersubstanzen wurden zwar die toxikologischen Probleme gemindert, aber diese Verbindungen können in färberischer Hinsicht nicht völlig zufriedenstellen.

Es bestand daher die Aufgabe, Oxidationshaarfärbemittel auf der Basis einer gut färbenden, jedoch nicht sensibilisierenden und nicht mutagenen Entwicklersubstanz-Kupplersubstanz-Kombination zur Verfügung zu stellen.

Hierzu wurde nun überraschend gefunden, daß durch ein Mittel zur oxidativen Färbung von Haaren mit einem Gehalt einer Kombination aus 2-(2,5-Diaminophenyl)ethanol als Entwicklersubstanz und mindestens einer Kupplersubstanz ausgewählt aus 3,4-Methylendioxyphenol, 3-Aminophenol und N-(2-Hydroxyethyl)-3,4-methylendioxyanilin die gestellte Aufgabe in hervorragender Weise gelöst wird.

Das 2-(2,5-Diaminophenyl)ethanol wird seit einiger Zeit als Bestandteil von Oxidationshaarfärbemitteln in der Patentliteratur erwähnt. Als möglicher Bestandteil von Oxidationshaarfärbemitteln ist es zum Beispiel in der eigenen europäischen Offenlegungsschrift 0 166 155 sowie in den eigenen deutschen Offenlegungsschriften 3 625 916, 3 627 398 und 3 706 565 sowie auch in der DE-OS 3 545 371 aufgeführt. Ferner fällt das 2-(2,5-Diaminophenyl)ethanol unter eine allgemeine Formel für 1-Hydroxyalkyl-2,5-diaminobenzolverbindengen, welche als Entwicklersubstanzen für Oxidationshaarfärbemittel in der eigenen europäischen Offenlegungsschrift 0 007 537 empfohlen werden. Die dort mehrfach konkret genannte Verbindung 1-Hydroxymethyl-2,5-diaminobenzol (2,5-Diaminobenzylalkohol) hat sich jedoch als sensibilisierend erwiesen. Die ausgezeichnete Eignung der Entwicklersubstanz 2-(2,5-Diaminophenyl)ethanol in Kombination mit den vorstehend genannten Kupplersubstanzen zur Lösung der gestellten Aufgabe wurde bisher nicht erkannt. Dementsprechend sind die erfindungsgemäßen Entwicklersubstanz-Kupplersubstanz-Kombinationen in keiner dieser Druckschriften beschrieben.

Die konkrete Darstellung des 2-(2,5-Diaminophenyl)ethanols ist bisher ebenfalls weder beschrieben noch hat diese Entwicklersubstanz Eingang in die Haarfärbepraxis gefunden. Wegen der inzwischen festgestellten sensibilisierenden Eigenschaften der aus der europäischen Offenlegungsschrift 0 007 537 bekannten homologen Vebindung 2,5-Diaminobenzylalkohol mußte eine Synthese dieser Verbindung auch nicht lohnend erscheinen.

Die Verwendung der Verbindung 2-(2,5-Diaminophenyl)ethanol als Entwicklersubstanz zeigt bezüglich der erzielbaren Färbungen keine Unterschiede gegenüber den üblicherweise eingesetzten Entwicklersubstanzen 2,5-Diaminotoluol und p-Phenylendiamin sowie dem 2,5-Diaminobenzylalkohol. Mit den erfindungsgemäßen Kupplersubstanzen werden die erwarteten Farbtöne erhalten. Die Echtheiten und die Stabilität der Färbungen sind vergleichbar mit den Echtheiten, die bei der Verwendung von 2,5-Diaminobenzylalkohol erzielt werden. Bezüglich der Intensitäten der erhaltenen Färbungen ist sogar ein geringfügig schlechteres Ergebnis festzustellen.

Der große und überraschende technische Fortschritt, der bei der Verwendung von 2-(2,5-Diaminophenyl)ethanol mit den vorstehend genannten Kupplersubstanzen erzielt werden kann, liegt vielmehr in den physiologischen Eigenschaften begründet. So zeigt das 2-(2,5-Diaminophenyl)ethanol im Mutagenitätstest nach Ames sowohl in Anwesenheit als auch in Abwesenheit von Wasserstoffperoxid, im Chromosomenaberrationstest an CHO-Zellen in vitro (CA-Test), im Mouse Lymphoma Test (Punktmutation in vitro) und im Sister-Chromatide-Exchange-Test (SCE-Test) keinerlei Mutagenität, während die Entwicklersubstanz 2,5-Diamino-benzylalkohol in einigen dieser Teste ein mutagenes Potential aufweist.

Ein weiterer Vorteil der erfindungsgemäßen Entwicklersubstanz-Kupplersubstanz-Kombination wird offenkundig, wenn die Entwicklersubstanz-Kupplersubstanz-Kombination in Haarfärbegemischen unter Anwendungsbedingungen oder wenn gefärbte Haarsträhnen im Amestest auf mutagene Wirkung untersucht werden.

Der Vergleich von Haarfärbelösungen, die die Entwicklersubstanz p-Phenylendiamin, 2,5-Diaminotoluol oder 2-(2,5-Diaminophenyl)ethanol enthalten, unter oxidativen Bedingungen im Amestest zeigt, daß die allgemein verwendeten Entwicklersubstanzen ein mutagenes Potential zeigen, während die erfindungsgemäße Entwicklersubstanz 2-(2,5-Diaminophenyl)ethanol nicht mutagen wirkt.

Ein weiterer, überraschender Vorteil für die erfindungsgemäße Entwicklersubstanz/Kupplersubstanz-Kombinationergibtsichaus dem Vergleich von gefärbten Haarsträhnchen im Amestest. Für diesen Versuch wurden Haarsträhnchen verwendet, die einerseits mit einer Mischung aus p-Phenylendiamin oder 2,5-Diaminotoluol als Entwiklersubstanz und verschiedenen Kupplerubstanzen und andererseits mit einer Mischung aus dem erfindungsgemäßen 2-(2,5-Diaminophenyl)ethanol und den gleichen Haarfarbkupplern eingefärbt worden waren.

Die Ergebnisse dieser Versuche zeigt die nachfolgende Tabelle 1:

Tabelle 1:

| Amesteste von gefärbten Haarsträhnen | | | |
|---|---|---|---|
| Kupplersubstanz | Entwicklersubstanz | | |
| | 2,5-Diamino-toluol | p-Phenylen-diamin | 2-(2,5-Diaminophenyl)-ethanol |
| ohne Kuppler-substanz | + | + | - |
| Resorcin | + | (+) | - |
| 2-Methyl-resorcin | + | + | - |
| 3,4-Methylendioxyphenol | + | + | - |
| 3-Aminophenol | (+) | (+) | - |
| + mutagen | (+) schwach mutagen | | -nicht mutagen |

Versuchsbedingungen: Haarsträhnchen aus 30-50 Haaren werden mit einem Mittel nach Beispiel 1, bei dem die Kupplersubstanz jeweils im äquimolaren Verhältnis zur Entwicklersubstanz enthalten war, getränkt.

Die Strähnen wurden anschließend 30 Minuten lang bei 40 Grad Celsius im Klimaschrank aufbewahrt, sodann gründlich mit Wasser gespült und schließlich getrocknet. Die so gefärbten Haare wurden nun in 2,5 cm lange Stücke geschnitten und nach der Methodik von B.N. Ames an Salmonella typhimurium-Stämmen TA97, TA98 und TA100 in Anwesenheit von S9-Mix geprüft.

Wie die Ergebnisse zeigen, ist im Gegensatz zu Kombinationen aus 2,5-Diaminotoluol oder p-Phenylendiamin und den vorstehend genannten Kupplersubstanzen kein mutagenes Potential der mit der erfindungsgemäßen Kombination gefärbten Haarsträhnchen vorhanden.

Weiterhin zeigt die erfindungsgemäße Entwicklersubstanz-Kupplersubstanz-Kombination in einem Sensibilisierungstest gemäß den OECD-Richtlinien sowohl bei topischer als auch bei intradermaler Behandlung keine sensibilisierende Wirkung. Dieser überraschende Befund ermöglicht es erstmals, Haarfärbemittel für Paraallergiker bereitzustellen. Mit den zum Stand der Technik gehörenden Entwicklersubstanz-Kupplersubstanz-Kombinationen ist dagegen die Herstellung von qualitativ hochwertigen, nicht mutagenen und nicht sensibilisierenden Haarfärbemitteln nicht möglich.

Der Gegenstand der vorliegenden Anmeldung betifft daher ein Mittel zur oxidativen Färbung von Haaren mit einem Gehalt an einer Entwicklersubstanz-Kupplersubstanz-Kombination, dadurch gekennzeichnet, daß es als Entwicklersubstanz 2-(2,5-Diaminophenyl)ethanol oder dessen physiologisch verträgliches, wasserlösliches Salz enthält, und die Kupplersubstanz ausgewählt ist aus 3,4-Methylendioxyphenol, 3-Aminophenol und N-(2-Hydroxyethyl)-3,4-methylendioxyanilin oder deren physiologisch verträglichen wasserlöslichen Salzen.

Die Entwicklersubstanzen und Kupplersubstanzen sollen darin als solche oder in Form der physiologisch verträglichen Salze, beispielsweise der Hydrochloride oder der Sulfate, enthalten sein.

Die erfindungsgemäßen Haarfärbemittel sollen das 2-(2,5-Diaminophenyl)ethanol oder dessen physiologisch verträgliche Salze in einer für die Haarfärbung ausreichenden Menge von 0,01 bis 4,0 Gewichtsprozent, vorzugsweise 0,1 bis 3,0 Gewichtsprozent enthalten, die Gesamtmenge der Kupplersubstanzen soll ebenfalls 0,01 bis 4,0 Gewichtsprozent, vorzugsweise 0,1 bis 3,0 betragen.

Aufgrund der vorteilhaften Eigenschaften des erfindungsgemäßen 2-(2,5-Diaminophenyl)ethanols soll diese Verbindung als einzige Enwicklersubstanz in den Mitteln enthalten sein.

Neben den vorstehend genannten erfindungsgemäß verwendeten Kupplersubstanzen können zur Erzielung gewisser Farbnuancen gegebenenfalls zusätzlich noch weitere Kupplersubstanzen in geringerer Menge enthalten sein, sofern daduch keine mutagene oder sensibilisierende Wirkung des Mittels auftritt. Vorzugsweise enthält das Mittel jedoch ausschließlich eine oder mehrere der vorstehend genannten Kupplersubstanzen. Die Kupplersubstanzen können in dem Haarfärbemittel jeweils einzeln oder im Gemisch miteinander enthalten sein. Die Gesamtmenge der in dem hier beschriebenen Haarfärbemittel enthaltenen Entwickler- und Kupplersubstanzen soll 0,01 bis 8,0 Gewichtsprozent, vorzugsweise 0,5 bis 6,0 Gewichtsprozent betragen.

Die Entwicklersubstanzen werden im allgemeinen in etwa äquimolaren Mengen bezogen auf die Kupplersubstanzen eingesetzt. Es ist jedoch nicht von Nachteil, wenn die Entwicklersubstanz in einem gewissen Überschuß oder Unterschuß vorhanden ist.

Weiterhin kann das Haarfärbemittel dieser Anmeldung zusätzlich andere nicht sensibilisierende und nicht mutagene direktfärbende Farbkomponenten, beispielsweise 4-(N-Ethyl,N-2'-hydroxyethyl)amino-1-(2''-hydroxyethyl)-amino-2-nitrobenzol und 1-(2'-Ureidoethyl-amino)-4-nitrobenzol, enthalten, wobei die Einsatzmenge dieser Farbstoffe etwa 0,1 bis 4,0 Gewichtsprozent beträgt.

Selbstverständlich können die Kuppler- und Entwicklersubstanzen sowie die anderen Farbkomponenten, sofern es sich um Basen handelt, in Form der kosmetisch verträglichen Säureadditionssalze, beispielsweise als Hydrochlorid oder als Sulfat oder - sofern es sich m Verbindungen mit Phenol- oder Säuregruppen handelt - in Form der Salze mit Basen, zum Beispiel als Alkaliphenolate, eingesetzt werden.

Die erfindungsgemäße Entwicklersubstanz 2-(2,5-Diaminophenyl)ethanol kann ausgehend von 2-(2-Aminophenyl)ethanol hergestellt werden. Dabei wird die aus 2-(2-Aminophenyl)ethanol und Essigsäureanhydrid erhältliche Diacetylverbindung nitriert. Die erhaltene Mischung von N-Acetyl-2-(2-acetoxyethyl)-4-nitroanilin und N-Acetyl)-2-(2-acetoxyethyl)-5-nitroanilin wird mit Salzsäure verseift. Das gewünschte 2-(2-Amino-5-nitrophenol)ethanol kann daraus durch partielle Hydrolyse der Hydrochloride abgetrennt werden. Das 2-(2,5-Diaminophenyl)ethanol wird daraus duch katalytische Hydrierung erhalten.

In der nachstehenden Beispielen soll der Gegenstand der Erfindung näher erläutert werden, ohne ihn auf die Beispiele zu beschränken.

## Beispiele für Haarfärbemittel

Beispiel 1: Haarfärbemittel in Cremeform

| | |
|---|---|
| 3,0 g | 2-(2,5-Diaminophenyl)ethanol-sulfat |
| 0,6 g | Resorcin |
| 0,6 g | 3-Amionphenol |
| 0,6 g | N-(2-Hydroxyethyl)-3,4-methylendioxy-anilin-hydrochlorid |
| 15,0 g | Cetylalkohol |
| 0,3 g | Natriumsulfit, wasserfrei |
| 3,5 g | Natriumlaurylalkohol-diglykolethersulfat, 26-prozentige wäßrige Lösung |
| 3,0 g | Ammoniak, 25-prozentige wäßrige Lösung |
| 73,4 g | Wasser |
| 100,0 g | |

50 g dieses Haarfärbemittels werden kurz vor dem Gebrauch mit 50 g Wasserstoffperoxidlösung (6-prozentig) vermischt. Anschließend trägt man das Gemisch auf ergrautes Haar von Menschen auf, die auf p-Phenylendiamin und p-Toluylendiamin sensibilisiert sind und läßt es 30 Minuten lang bei 40 Grad Celsius einwirken. Danach wird mit Wasser gespült und getrocknet. Das Haar ist schwarz gefärbt. Die Probanden (Paraallergiker) reagieren während und nach der Färbung nicht allergisch.

Beispiel 2: Haarfärbelösung

| 1,25 g | 2-(2,5-Diaminophenyl)ethanol-sulfat |
|---|---|
| 1,10 g | N-(2-Hydroxyethyl)-3,4-methylen-dioxyanilinhydrochlorid |
| 10,00 g | Natriumlaurylalkohol-diglykolether-sulfat, 28-prozentige wäßrige Lösung |
| 10,00 g | Ammoniak |
| 77,65 g | Wasser |
| 100,00 g | |

Man vermischt kurz vor dem Gebrauch 10 g des vorstehenden Haarfärbemittels mit 10 g Wasserstoffperoxid (6-prozentig) und läßt die Mischung 30 Minuten lang bei 40 Grad Celsius auf blonde Naturhaare einwirken. Nach dem Spülen mit Wasser und dem Trocknen ist das Haar in einem grünlich aschigen Naturton gefärbt.

**Patentansprüche**

1. Mittel zur oxidativen Färbung von menschlichen Haaren mit einem Gehalt an einer Entwicklersubstanz-Kupplersubstanz-Kombination, **dadurch gekennzeichnet**, dass es als einzige Entwicklersubstanz 2-(2,5-Diaminophenyl)ethanol oder dessen physiologisch, verträgliches, wasserlösliches Salz enthält und mindestens eine Kupplersubstanz enthält, die ausgewählt ist aus 3,4-Methylendioxy-phenol, 3-Aminophenol und N-(2-Hydroxyethyl)-3,4-methylendioxyanilin oder deren physiologisch verträglichen, wasserlöslichen Salzen.

2. Mittel nach Anspruch 1, **dadurch gekennzeichnet**, dass die Entwicklersubstanz-Kupplersubstanz-Kombination in einer Menge von 0,01 bis 8,0 Gewichtsprozent, vorzugsweise 0,5 bis 6,0 Gewichtsprozent, enthalten ist.

3. Mittel nach einem der Ansprüche 1 oder 2, **dadurch gekennzeichnet,** dass es eine Farbkomponente enthält, welche ausgewählt ist aus 4-(N-Ethyl,N-2'-hydroxyethyl)amino-1-(2'-hydroxyethyl)amino-2-nitrobenzol und 1-(2'-Ureidoethylamino)4-nitrobenzol.

4. Mittel nach einem der **Ansprüche 1** bis 3, **dadurch gekennzeichnet**, dass es als Antioxidantien Ascorbinsäure, Thioglykolsäure oder Natriumsulfit enthält.

5. Mittel nach einem der Ansprüche 1 bis 4, **dadurch gekennzeichnet**, dass es einen pH-Wert von 8,0 bis 11,0 aufweist.

6. Verwendung eines Mittels zur oxidativen Färbung von menschlichen Haaren mit einem Gehalt an einer Entwicklersubstanz-Kupplersubstanz-Kombination, **dadurch gekennzeichnet**, dass es als einzige Entwicklersubstanz 2-(2,5-Diaminophenyl)ethanol oder dessen physiologisch verträgliches, wasserlösliches Salz enthält und mindestens eine Kupplersubstanz enthält, die ausgewählt ist aus 3,4-Methylendioxyphenol, 3-Aminophenol und N-(2-Hydroxyethyl)-3,4-methylendioxyanilin oder deren physiologisch verträglichen, wasserlöslichen Salzen, zur Färbung der Haare von Paraallergikern.

**Claims**

1. Agent for the oxidative colouring of human hair with a content of a developer-coupler substance combination, characterized in that it contains as sole developer substance 2-(2,5-diamino - phenyl)ethanol or a physiologically-compatible, water-soluble salt thereof, and at least one coupler substance selected from 3,4-methylenedioxyphenol, 3-amino-phenol and N-(2-hydroxy - ethyl)-3,4-methylenedioxyaniline or physiologically-compatible salts thereof.

2. Agent according to claim 1, characterized in that the developer-coupler substance combination is present in an amount of 0,01 to 8,0% by weight, preferably 0,5 to 6,0 % by weight.

3. Agent according to any of claims 1 to 2, characterized in that it contains a colouring component, which is selected from 4-(N-ethyl, N-2'-hydroxyethyl)-amino-1-(2''-hydroxyethyl)-amino-2-nitrobenzene and 1-(2'-ureidoethyl-amino)-4-nitrobenzene.

4. Agent according to any of claims 1 to 3, characterized in that it contains ascorbic acid, thioglycolic acid or sodium sulphite as antioxidans.

5. Agent according to any of claims 1 to 4, characterized in that it has a pH of 8,0 to 11,0.

6. Use of an agent for the oxidative colouring of human hair with a content of a developer-coupler substance combination characterized in that it contains as sole developer substance 2-(2,5-diaminophenyl)ethanol or a physiologically-compatible, water-soluble salt thereof and at least one coupler substance selected from 3,4-methylenedioxyphenol, 3-aminophenol and N-(2-hydroxyethyl)-3,4-methylenedioxyaniline or physiologically-compatible salts thereof, for the colouring of hairs of para-allergic persons.

**Revendications**

1. Agent pour la teinture oxydante de cheveux humains présentant une teneur en une combinaison d'un agent développeur - agent coupleur caractérisé en ce qu'il contient en tant qu'agent développeur unique du 2-(2,5-diaminophényl)éthanol ou son sel hydrosoluble physiologiquement compatible et au moins un agent coupleur sélectionné parmi le 3,4-méthylénedioxyphénol, le 3-aminophénol et la N-(2-hydroxyéthyl)-3,4-méthylènedioxyaniline ou leurs sels hydrosolubles physiologiquement compatibles.

2. Agent selon la revendication 1, caractérisé en ce que la combinaison d'un agent développeur - agent coupleur est comprise dans une quantité allant de 0,01 à 8,0 pour-cent en masse, de préférence de 0,5 à 6,0 pour-cent en masse.

3. Agent selon l'une des revendications 1 ou 2, caractérisé en ce qu'il contient une composante colorante qui est sélectionnée parmi le 4-(N-éthyl, N-2'-hydroxyéthyl)amino-1-(2'-hydroxyéthyl)amino-2-nitrobenzène et le 1-(2'-uréidoéthylamino)4-nitrobenzène.

4. Agent selon l'une des revendications 1 à 3, caractérisé en ce qu'il contient en tant qu'antioxygénes de l'acide ascorbique, de l'acide thioglycolique ou du sulfite de sodium.

5. Agent selon l'une des revendications 1 à 4, caractérisé en ce qu'il présente une valeur de pH comprise entre 8,0 et 11,0.

6. Utilisation d'un agent pour une teinture oxydante de cheveux humains présentant une teneur en une combinaison d'un agent développeur - agent coupleur, caractérisée en ce que l'agent contient en tant qu'agent développeur unique du 2-(2,5-diaminophényl)éthanol ou son sel hydrosoluble physiologiquement compatible et au moins un agent coupleur sélectionné parmi le 3,4-méthylènedioxyphénol, le 3-aminophénol et la N-(2-hydroxyéthyl)-3,4-méthylènedioxyaniline ou leurs sels hydrosolubles physiologiquement compatibles, afin de colorer les cheveux de para-allergiques.